# EUROPEAN PATENT APPLICATION

(11) **EP 2 896 688 A1**
(43) Date of publication of application: **22.07.2015**
(21) Application number: 14305075.5
(22) Date of filing: 20.01.2014
(51) Int. Cl.: C12N 5/071

(54) **A method of producing beta pancreatic cells from progenitor cells through the use of hydrogen peroxide**

(71) Applicant: Centre National de la Recherche Scientifique (CNRS), 75016 Paris (FR); UNIVERSITE PARIS DESCARTES, 75006 Paris (FR); INSTITUT NATIONAL DE LA SANTE ET DE LA RECHERCHE MEDICALE (INSERM), 75013 Paris (FR)
(72) Inventor: Duvillié, Bertrand, 92120 Montrouge (FR); Hoarau, Emmanuelle, 75013 Paris (FR)
(74) Representative: Regimbeau

(57) **Abstract**

The inventors have surprisingly found that it is possible to increase the ratio of beta cells and progenitors thereof, obtained from undifferentiated cells by manipulation of culture conditions. In particular, they found that the use of hydrogen peroxide (H₂O₂) at appropriate concentrations, in the culture medium of culture of embryonic pancreases containing stem cells, can significantly increase the ratio of beta cells obtained from stem cells.The present invention relates to an *in vitro* method for obtaining cells of the pancreatic endocrine lineage, and in particular of endocrine islets cells or precursor thereof, from undifferentiated cells, such as embryonic stem cells or induced pluripotent cells. The invention further encompasses cells of the pancreatic endocrine lineage obtainable by the present methods, compositions comprising said cells, and applications thereof.

## Description

The present invention relates to an *in vitro* method for obtaining cells of the pancreatic endocrine lineage, and in particular of endocrine islets cells or precursor thereof, from undifferentiated cells, such as embryonic stem cells or induced pluripotent cells. The invention further encompasses cells of the pancreatic endocrine lineage obtainable by the present methods, compositions comprising said cells, and applications thereof.

Diabetes mellitus is characterized by either the insensitivity to insulin secreted by the body (type II diabetes) and/or the inability to produce insulin (type I diabetes). In either case, the body is unable to lower glucose levels. This leads to a variety of local and systemic detrimental effects.

Type I diabetes is caused by the autoimmune destruction of the pancreatic islet insulin-producing beta cells. Insulin administration does not prevent the long-term complications of the disease, and replacement of the damaged cells with regulated insulin-producing cells is considered the ultimate cure for this type of diabetes.

Pancreas engraftment has been successful but is severely limited by the shortage of donors.

The need for developing alternatives to human pancreas donors, namely abundant sources of insulin-producing cells, has promoted the development of improved methods for obtaining beta cells of an appropriate quality and quantity for engraftment.

Terminally differentiated islet cells are difficult to expand in tissue culture. One favored approach is thus the generation of beta cells from stem cells, such as, for example, embryonic stem cells or induced pluripotent cells.

In vertebrate embryonic development, a pluripotent cell gives rise to a group of cells comprising three germ layers (ectoderm, mesoderm, and endoderm) in a process known as gastrulation. The pancreas will develop from the endoderm. Endoderm cells express a number of markers, such as, HNF3beta, GATA4, MIXL1, CXCR4 and SOX17. Formation of the pancreas arises from the differentiation of definitive endoderm into pancreatic endoderm (Annu Rev Cell Dev Biol, 25:221-251, 2009; Dev Dyn, 238:29-42, 2009). Pancreatic tissues comprising endocrine cells, among which beta cells, arise from the differentiation of pancreatic endoderm.

Due to the need for beta cell replacement therapy, much work has been done in the past decade to generate beta cells from a variety of stem cell sources (Weir et al., Genome Medicine; 3:61; 2011). Several methods have been developed to obtain beta cells from stem cells, such as for example the methods described in D'Amour et al. (Nat Biotechnol., (11):1392-401, 2006).

However, in this method, the maturity, specificity and function of the beta cells are not satisfying.

Thus, there is still a need for improved methods for obtaining beta cells, particularly in order to increase the yield of beta cells that can be obtained with them.

### Description

The inventors have surprisingly found that it is possible to increase the ratio of beta cells and progenitors thereof, obtained from undifferentiated cells by manipulation of culture conditions. In particular, they found that the use of hydrogen peroxide (H₂O₂) at appropriate concentrations, in the culture medium of culture of embryonic pancreases containing stem cells, can significantly increase the ratio of beta cells obtained from stem cells. Interestingly, they found that this result could also be obtained when using glucose oxidase that catalyzes the conversion of glucose into gluconolactone and hydrogen peroxyde, which acts as a precursor of H2O2.

It is considered in the art that insulin-producing beta cells have a low anti-oxydative defense status and exceptional susceptibility toward free radical-induced oxidative damage (Mechlovichet al., J Pharmacol Exp Ther.; 333(3):874-82; 2010).

Thus, one skilled in the art would not have been incited to use reactive oxygen species such as H₂O₂ in a cell culture intended for beta cells.

Interestingly, the concentrations of H₂O₂ used did not produce detectable oxidative stress or damage in the resulting beta cells. The cells obtained can advantageously be used in therapeutic treatments, in particular in the case of beta cells obtained from human stem cells.

The cells of the invention can further be used for the *in vitro* production of insulin.

### Figure legends

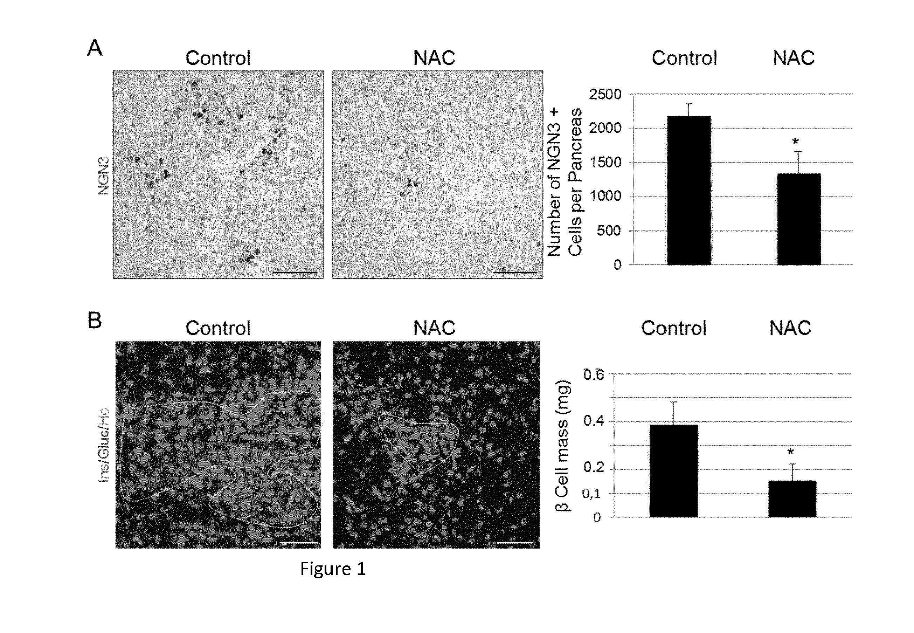

Figure 1. Antioxidant NAC decreases beta cell differentiation *in vivo*
   A: Pregnant female rats were treated with 10 mM NAC from E13.5. Embryonic pancreases were analyzed at E20.5 A : NGN3 expression (in black) was detected by immunohistochemistry and the number of NGN3-positive cells was quantified. B : Islets were detected in embryonic pancreases from both conditions, using anti-insulin and anti-glucagon antibodies. Next, beta cell mass was calculated. Each point represents the mean ± SEM of three individual data pools. *P <0.05. Scale bar : 10 µm.

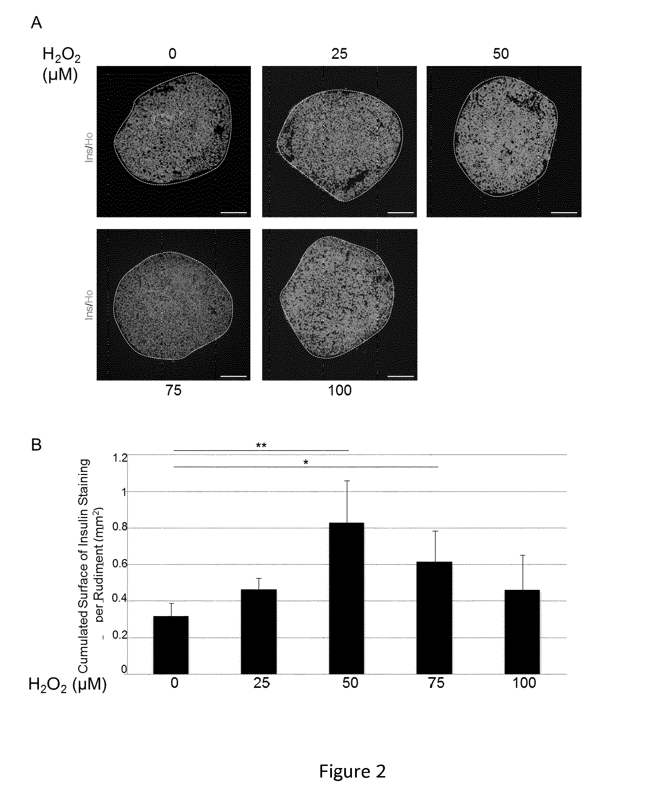

Figure 2. Effect of H₂O₂ on beta cell development.
   A : E13.5 rat pancreases were cultured for 7 days with H₂O₂.(O-100 µM). At day 7, anti-insulin antibody was used to detect beta cell development. B : The absolute surface area occupied by insulin-positive cells was quantified. Each point represents the mean ± SEM of three individual data pools. *P <0.05; **P < 0.01. Scale bar : 50 µm.

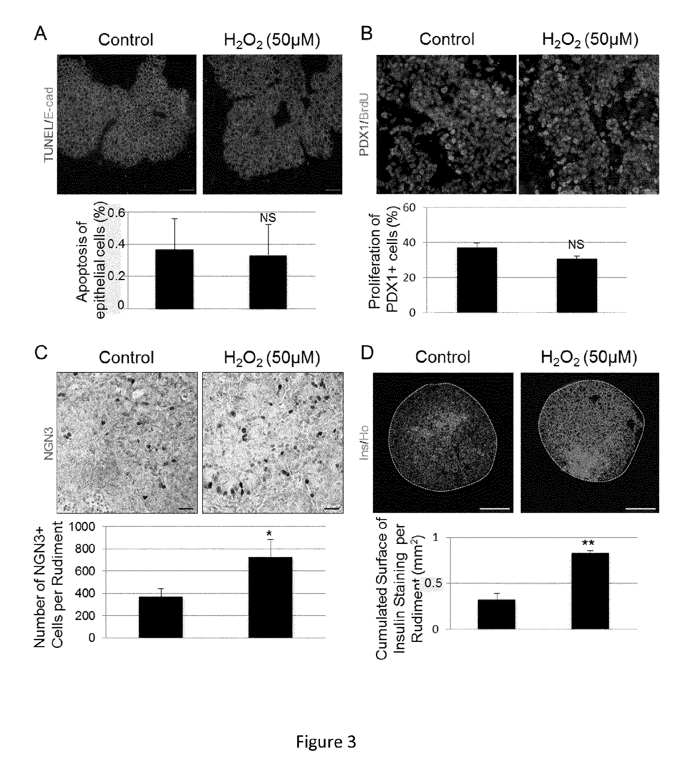

Figure 3. H₂O₂ induces beta cell differentiation
   A : Apoptotic cells were detected using a TUNEL reaction assay in pancreases cultured for 1 day with or without 50 µM H₂O₂. The epithelial cells were detected using an anti-E-cadherin antibody. Note the absence of apoptotic cells within the epithelium. Next, apoptosis percentage was calculated. B : Proliferation of early PDX1+ progenitors was observed in pancreases cultured for 1 day with or without 50 µM H₂O₂ (both PDX1 and BrdU staining appear on the picture). BrdU was added 1 hour before the end of the culture. Proliferation percentage was also quantified. C : At day 1, NGN3 expression (in black) was detected by immunohistochemistry. For each pancreas, the number of NGN3+ cells was quantified. D : At day 7, immunohistochemistry was performed to detect insulin. The insulin staining surface was measured in both conditions. Each point represents the mean ± SEM of three individual data pools. *P <0.05; **P < 0.01. Scale bar : 50 µm.

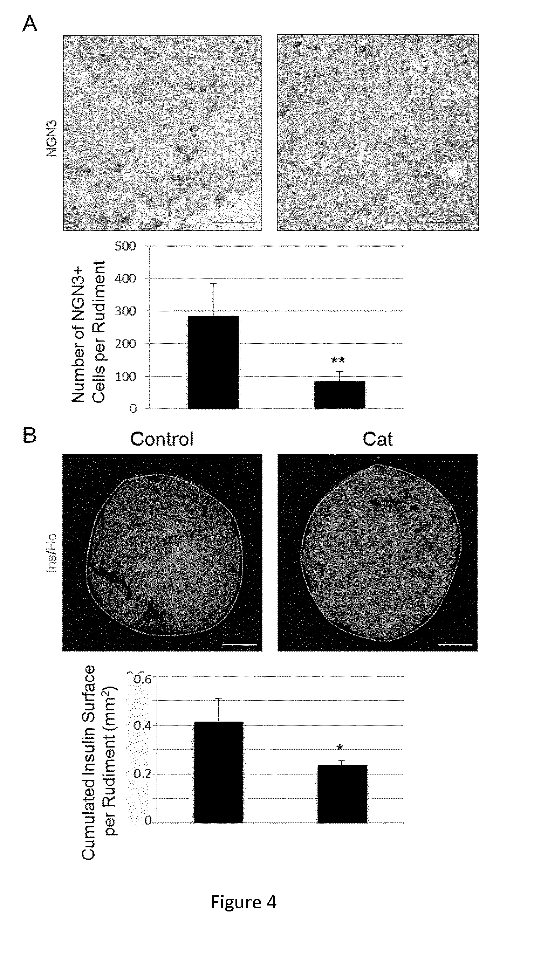

Figure 4. Exogenous Catalase reduces beta cell differentiation
   E13.5 pancreases were cultured for 1 to 7 days with or without Catalase at 300U/mL. A : After 1 day of culture, NGN3 expression was detected by immunohistochemistry (in black). The absolute number of NGN3+ cells was quantified. B : After 7 days, beta cell area was detected by immunohistochemistry and quantified. Each point represents the mean ± SEM of three individual data pools. *P <0.05; **P < 0.01. Scale bar : 50 µm.

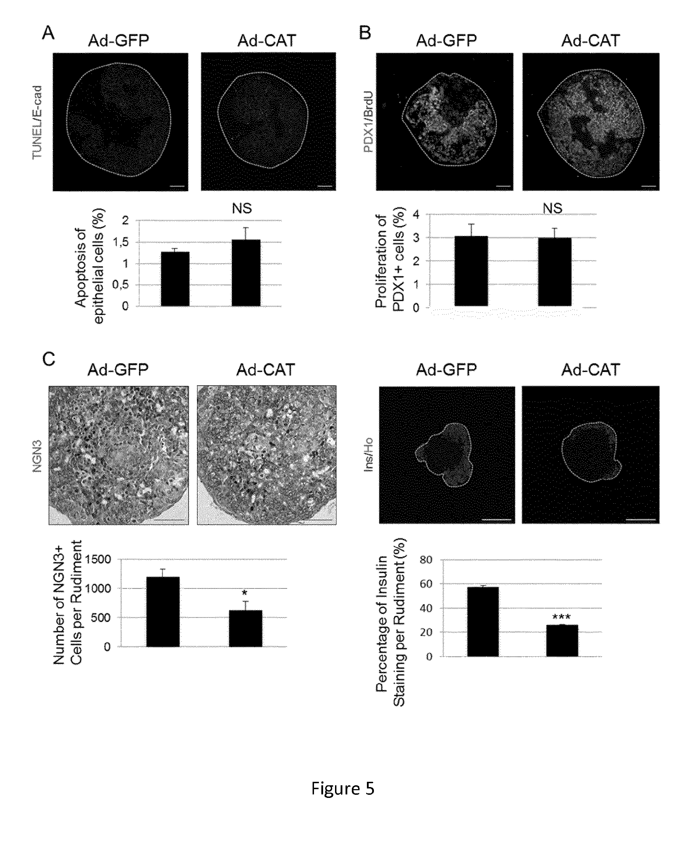

Figure 5. Catalase overexpression decreases the number of NGN3-positive cells in the pancreatic epithelium.
   E13.5 rat pancreatic epithelia were infected by adenovirus coding either for GFP (controls) either for catalase and cultured for 1 or 3 days. A : At day 3, NGN3 expression (in black) was checked by immunohistochemistry and the number of NGN3+ cells per epithelium was quantified. B : Proliferation of early PDX1+ progenitors was detected by immunohistochemistry (PDX1 and BrdU staining both appear on the picture). Proliferation percentage was quantified. C : Apoptotic cells were detected using a TUNEL reaction assay. The epithelial cells were detected using an anti-E-cadherin antibody. Next, apoptosis percentage of epithelial cells was calculated. Each point represents the mean ± SEM of three individual data pools. *P <0.05; **P < 0.01. Scale bar : 50 µm.

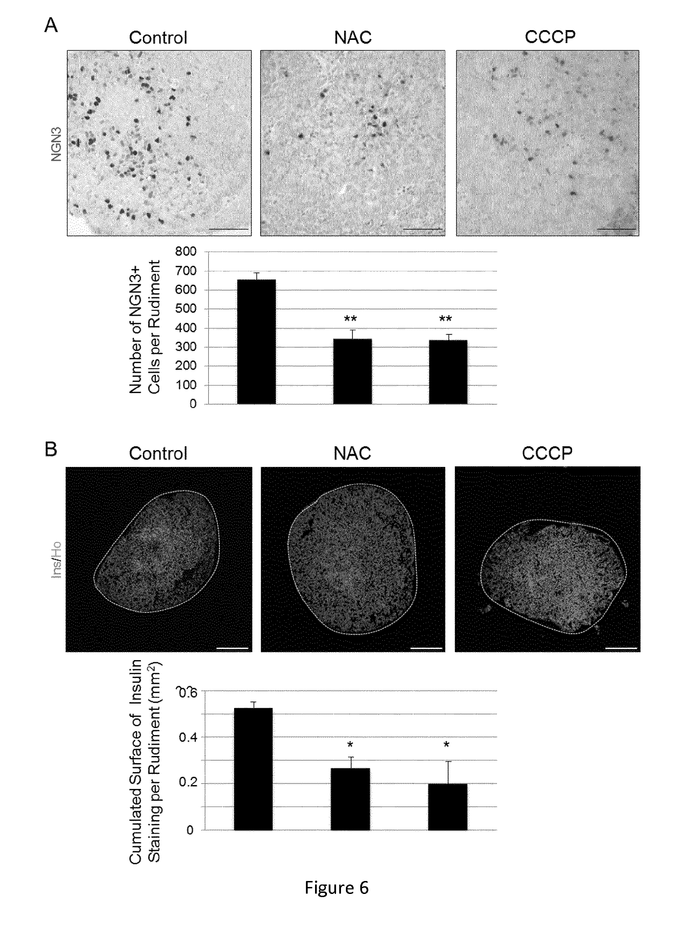

Figure 6. N-AcetylCystein and the mitochondrial agent CCCP alter beta-cell differentiation
   E13.5 rat pancreases were cultured for 1 and 7 days with or without NAC at 10 µM or CCCP at 1 µM. A : After 1 day, NGN3 expression (in black) was detected by immunohistochemistry. The number of NGN3+ cells per epithelium was quantified. B : At day 7, beta cell area was detected by immunohistochemistry and quantified. Each point represents the mean ± SEM of three individual data pools. *P <0.05; **P < 0.01. Scale bar : 50 µm.

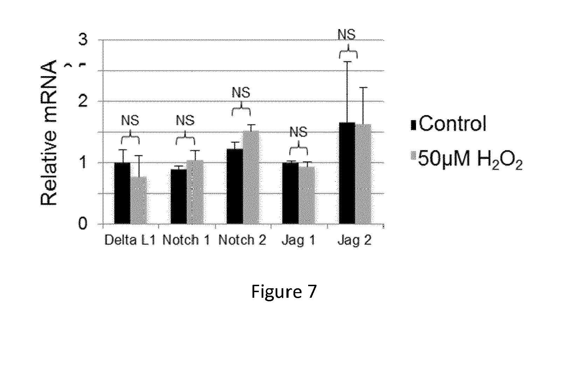

Figure 7. Notch Pathway is not implicated in the activation of beta cell development by H₂O₂
   Pancreases were cultured one day with or without 50 µM H₂O₂. Expression of genes encoding the Notch receptors 1 and 2, the Notch target Hes 1, the Notch ligands Jagged 1, Jagged 2, Delta-like 1 were analyzed by RT-qPCR. Gene expression is presented as a percentage of the highest sample. Each point represents the mean ± SEM of three individual pools.

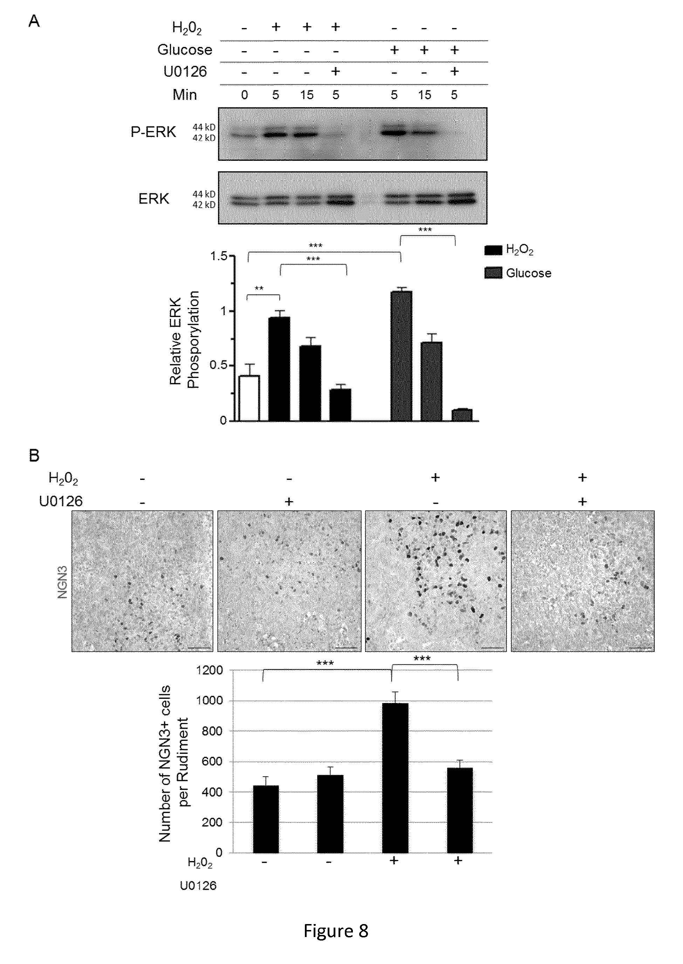

Figure 8. ERK Phosphorylation by H₂O₂ is required for the proper development of beta cells
   A : E13.5 rat pancreases were cultured for 0, 5 or 15 min with or without 50 µM H₂O₂ and with or without 20mM Glucose. Protein extracts from pancreases cultured were analyzed by Western blot to quantify ERK1/2 and MEK Phosphorylation. Total ERK and beta-actin are used as loading control. B : E13.5 rat pancreases were cultured with or without H₂O₂ at 50 µM, in association with the ERK1/2 inhibitor U0126. For each condition, NGN3 expression (in black) was detected by immunohistochemistry and the number of NGN3+ cells was quantified. Each point represents the mean ± SEM of three individual data pools. *P <0.05; **P < 0.01.

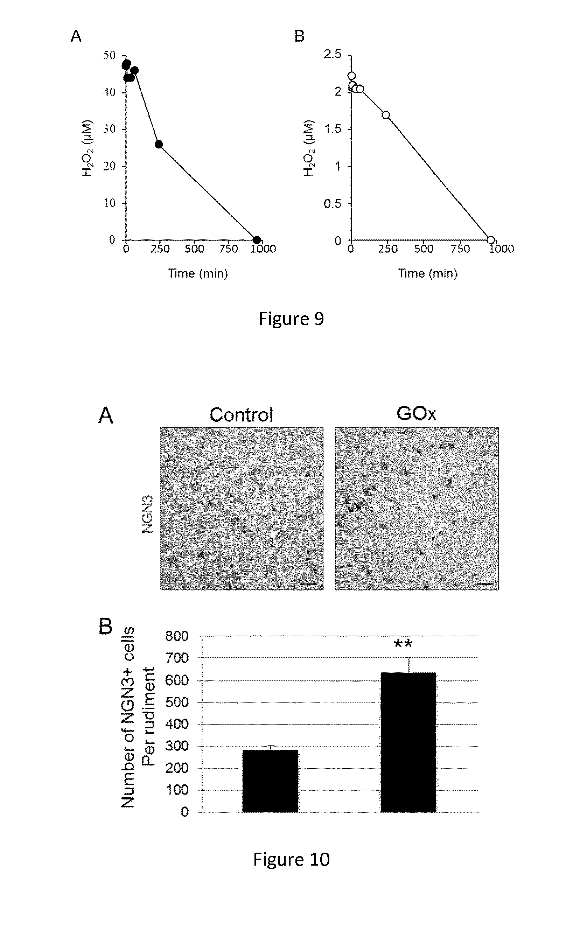

Figure 9. Kinetics of hydrogen peroxide decay at 37°C in culture medium
   Residual hydrogen peroxide (initial concentration: 50 µM, A; or 2.5 µM, B) was quantified by polarographic determination of the oxygen released upon addition of 10 µg of purified beef liver catalase resuspended in 50 mM phosphate buffer pH 7.0 (Sigma Chemical Co.) to 250 µl culture medium samples placed in an oxygen polarograph (Hansatech, Norfolk, England).
Figure 10. Glucose oxidase mimics the effect of H₂O₂
   Pancreases were cultured one day with GOx at 0,3U/mL. A : NGN3 expression (in black) was detected by immunohistochemistry. B : The number of NGN3-positive cell was then quantified for each rudiment. Each point represents the mean ± SEM of three individual data pools. **P < 0.01.
Figure 11. Infection of pancreatic epithelia with Ade-Cat induces efficient Catalase expression.
   Pancreatic epithelia were infected with adenoviruses coding either for GFP either for Catalase. A : After one day, Catalase was detected by immunohistochemistry. B : beta cell area was detected and quantified in both conditions. Each point represents the mean ± SEM of three individual data pools. **P < 0.01.

### Detailed description

Unless otherwise defined herein, scientific and technical terms used in connection with the present invention shall have the meanings that are commonly understood by those of ordinary skill in the art. Further, unless otherwise required by context, nomenclatures and techniques refer to those well-known and commonly used in the art.

Nevertheless, with respect to the use of different terms throughout the current specification, the following definitions more particularly apply.

According to the invention, the terms "comprising" or "containing" mean, without limitation, the inclusion of the referent and do not exclude the presence of other elements. For example, "a method comprising the step of x" encompasses any method in which x is carried out, independently of the fact that additional steps are also performed. Likewise, "a composition comprising x and y" encompasses any composition that contains x and y, no matter what other components may be present in the composition.

In contrast, the terms "consisting of" mean the inclusion of the referent and the exclusion of any element not explicitly listed. When referring to a method, the terms "consisting of the steps x, y, and z" encompass methods in which steps x, y and z are performed, and wherein non-listed steps are not.

The term "about" means that the value of reference can vary within a certain range depending on the margin of error allowed, which may be easily determined by one skilled in the art.

A first object of the invention is an *in vitro* method for obtaining cells of the pancreatic endocrine lineage from stem cells, comprising a step of culturing stem cells in a medium comprising H₂O₂ or a precursor thereof. Preferably, said stem cells exclude human embryonic stem cells isolated by techniques that involve the destruction of an embryo.

The invention also pertains to the *in vitro* use of H₂O₂ or a precursor thereof for obtaining cells of the pancreatic endocrine lineage from stem cells.

The person skilled in the art will easily understand that an *in vitro* method for obtaining cells of the pancreatic endocrine lineage from stem cells refers to a method for the *in vitro* differentiation of stem cells into cells of the pancreatic endocrine lineage.

According to the invention, the term "differentiation" refers to the process by which a cell acquires the features of a more differentiated (or "specialized") cell such as, for example, a nerve cell or a muscle cell. Thus, in the context of the invention, a differentiated cell or a differentiation-induced cell is one that has more specialized features, compared to an undifferentiated cell, wherein said features correspond to a more differentiated stage within the lineage of a cell.

According to the invention, the lineage of a cell encompasses all of the discrete development stages of a cell within a scheme of development, that is to say from an undifferentiated stage to a differentiated stage. In that regard, according to the invention, a lineage-specific marker refers to a marker specifically associated with the phenotype of cells of a lineage of interest and can be used to assess the differentiated status of a cell.

By **"stem cells",** it is herein referred to cells defined by their ability to divide and differentiate into more specialized cells. By "ability to differentiate", it is herein referred to the cells ability to acquire and maintain physiological traits of more specialized cells. Thus, preferably, by "stem cells" it is herein referred to cells that have self-renewal and potency properties.

The stem cells of the invention are able to produce progeny cells, including self-renewing progenitors, non-renewing progenitors, and terminally differentiated cells. The stem cells according to the invention are also characterized by their ability to differentiate *in vitro* into functional cells of various cell lineages.

According to the invention, the terms **"stem cells"** encompass embryonic stem cells, perinatal stem cell, somatic stem cells, and bioengineered stem cells.

According to the invention, the terms **"self-renewal property",** as regards a cell, refer to the ability for said cell to go through several cycles of cell division while still maintaining its undifferentiated state.

According to the invention, the terms **"potency property",** as regards a stem cell, refer to the ability to differentiate into at least one cell type. In the context of the invention, the terms "potency property" encompass totipotency, pluripotency, multipotency, oligopotency and unipotency properties.

According to the invention, the terms **"totipotency property",** as regards a cell, refer to the ability to differentiate into any cell type of an organism.

According to the invention, the terms **"pluripotency property",** as regards a stem cell, refer to the ability to differentiate into several distinct cell types of any of the three germ layers of an organism: endoderm (such as cells of the interior stomach lining, the gastrointestinal tract, or the lungs), mesoderm (such as cells of muscle, bone, blood, or urogenital tissues), or ectoderm (such as cells of the epidermal tissue or of the nervous system).

According to the invention, the terms **"multipotency property",** as regards a stem cell, refer to the ability to differentiate into multiple, but limited cell types of an organism. Preferably, the **"multipotency property"** refers to the ability to differentiate into multiple cell types from the same tissue. For example, a mammal multipotent blood stem cell is a hematopoietic cell - and this cell type can itself differentiate into several types of blood cell types like lymphocytes, monocytes, neutrophils, but cannot differentiate into brain cells, bone cells or other non-blood cell types. Human multipotent cells can be found, for example, in human in adipose tissue, among cardiac cells, in bone marrow, and among mesenchymal stromal cells (MSCs) which can for example be found in the third molar.

According to the invention, the terms **"oligopotency property",** as regards a stem cell, refer to the ability to differentiate into a few cell types. Examples of oligopotent stem cells are the myeloid or lymphoid stem cells. For example, a mammal lymphoid cell can differentiate into various blood cells such as B and T cells, however not to a different blood cell type like a red blood cell.

According to the invention, the terms **"unipotency property",** as regards a stem cell, refer to cells that are restricted to differentiate into only one cell type.

The potency of a cell can easily be assayed *in vitro,* using methods such as clonogenic assays, where the progeny of a single cell is characterized. Such assays are conventional techniques for the person skilled in the art, who can further refer to Franken et al. (Nat Protoc.; 1 (5):2315-9; 2006) or Bianco et al. (Cell Stem Cell.; 2(4):313-319; 2008) for detailed protocols.

In the context of the invention, the stem cells can be mammalian stem cells.

According to the invention, the terms **"mammalian stem cells"** encompass mammalian embryonic stem cells, mammalian perinatal stem cell, mammalian somatic stem cells, and mammalian bioengineered stem cells. Preferably, according to the invention, mammalian stem cells are chosen from the list consisting in mammalian embryonic stem cells, mammalian perinatal stem cell, mammalian somatic stem cells, and mammalian bioengineered stem cells.

By **"mammalian embryonic stem cells",** it is herein referred to **mammalian** stem cells derived from the inner cell mass (ICM) of a mammalian embryo at the blastocyst stage.

By **"embryo"** it is herein referred to a multicellular diploid eukaryote in its earliest stage of development, from the time of first cell division until birth, hatching, or germination.

By **"blastocyst"** it is herein referred to a structure formed in the early development of mammals. Typically, it possesses an inner cell mass (ICM), or embryoblast, which subsequently forms the embryo, and an outer layer of cells, or trophoblast, surrounding the inner cell mass and a fluid-filled cavity known as the blastocoele.

According to the invention, mammalian embryonic stem cells may be either obtained from an established cell line, or isolated from an embryo by different techniques.

Techniques for isolating a stem cell from an embryo are well known from the person skilled in the art, and include either technique that involve the destruction of an embryo, or techniques that do not involve the destruction of an embryo.

Briefly, conventional techniques for obtaining embryonic stem cells (which involve the destruction of an embryo) may comprise the steps of isolating a primate blastocyst, isolating cells from the inner cellular mass (ICM) of the blastocyst, plating the ICM cells on a fibroblast layer (wherein ICM-derived cell masses are formed) removing an ICM-derived cell mass and dissociating the mass into dissociated cells, replating the dissociated cells on embryonic feeder cells and selecting colonies with compact morphology containing cells with a high nucleus/cytoplasm ratio, and prominent nucleoli. The cells of the selected colonies are then cultured.

For example, conventional techniques for obtaining embryonic stem cells (which involve the destruction of an embryo) have been described in U.S. Patent No. 5,843,780, and by Thomson et al. (Science; 282: 1145-1147; 1998; Curr. Top. Dev. Biol; 38: 133; 1998; Proc. Natl. Acad. Sci. U.S.A., 92:7844; 1995).

Briefly, embryonic stem cells can be extracted from human embryos without resulting in embryo destruction, using a technique used in pre-implantation genetic diagnosis.

For example, a technique that does not involve the destruction of an embryo has been described by Chung et al. (Cell Stem Cell; 2(2):113-7; 2008).

In an embodiment, mammalian stem cells are human stem cells.

By **"human embryonic stem cells",** it is herein referred to human stem cells derived from the inner cell mass (ICM) of a human embryo at the blastocyst stage. Human embryos reach the blastocyst stage 4-5 days post fertilization, at which time they consist of between 50 and 150 cells. Embryonic stem cells are pluripotent stem cells.

According to the invention, human embryonic stem cells may be either obtained from an established cell line, or isolated from an embryo by different techniques known from the person skilled in the art.

By **"human embryo"** it is herein referred to a multicellular diploid eukaryote in its earliest stage of development, from the time of first cell division until about eight weeks after fertilization (or about ten weeks after the last menstrual period). By contrast, a multicellular diploid eukaryote after more than about eight weeks after fertilization and before birth is called a fetus.

Non-limiting examples of human embryonic stem cells lines are for example the cell lines CHB-1,CHB-2, CHB-3, CHB-4, CHB-5, CHB-6, CHB-8, CHB-9, CHB-10, CHB-11, CHB-12, Rockefeller University Embryonic Stem Cell Line 1 (RUES1), Rockefeller University Embryonic Stem Cell Line 2 (RUES2), HUES 1, HUES 2, HUES 3, HUES 4, HUES 5, HUES 6, HUES 7, HUES 8, HUES 9, HUES 10, HUES 11, HUES 12, HUES 13, HUES 14, HUES 15, HUES 16, HUES 17, HUES 18, HUES 19, HUES 20, HUES 21, HUES 22, HUES 23, HUES 24, HUES 26, HUES 27, HUES 28, CyT49, Rockefeller University Embryonic Stem Cell Line 3 (RUES3), WA01 (H1), UCSF4, NYUES1, NYUES2, NYUES3, NYUES4, NYUES5, NYUES6, NYUES7, HUES 48, HUES 49, HUES 53, HUES 65, HUES 66, UCLA 1, UCLA 2, UCLA 3, WA07 (H7), WA09 (H9), WA13 (H13), WA14 (H14), HUES 62, HUES 63, HUES 64, CT1, CT2, CT3, CT4, MA135, Endeavour-2, WIBR1, WIBR2, HUES 45, Shef 3, Shef 6, WIBR3, WIBR4, WIBR5, WIBR6, BJNhem19, BJNhem20, SA001, SA002, UCLA 4, UCLA 5, UCLA 6, HUES, ESI-014, ESI-017, WA15, WA17, WA18, WA19, WA20, WA21, WA22, WA23, WA24, CSES2, CSES4, CSES7, CSES8, CSES11, CSES12, CSES13, CSES14, CSES15, CSES17, CSES19, CSES20, CSES21, CSES22, CSES23, CSES24, CSES25, HAD-C 100, HAD-C 102, HAD-C 106, ESI-035, ESI-049, ESI-051, ESI-053, CSES5, CSES6, CSES18, CA1, CA2, MEL-1, MEL-2, MEL-3, MEL-4, UCLA 8, UCLA 9, UCLA 10, UM4-6, GENEA002, GENEA048, Elf1, HUES 42, HUES 44, NMR-1, UM14-1, UM14-2, HUES 68, HUES 70, HUES 69, HUES PGD 10, UCLA 11, UCLA 12, WA25, WA26, WA27, HS346, HS401, HS420, I3 (TE03), I4 (TE04), I6 (TE06), UM22-2, CR-4, KCL011, GENEA015, GENEA016, GENEA047, GENEA042, GENEA043, GENEA057, GENEA052.

For ethical reasons, the present invention preferably does not pertain to objects that may be considered as contrary to *"ordre public"* or morality. Therefore, in the context of the invention, the terms "human embryonic stem cells" preferably refer to human embryonic stem cells which isolation has not involved the destruction of an embryo. In other words, the terms "human embryonic stem cells" preferably exclude human embryonic stem cells isolated by techniques that involve the destruction of an embryo.

In the context of the invention, it is to be understood that any technique that does not involve the destruction of an embryo can be used, including those that are not described herein.

Moreover, in the context of the invention, the embryos used for obtaining human embryonic stem cells are preferably embryos that cannot give rise to a human being, such as embryos destined to be discarded following *in vitro* fertilization (IVF) and embryos created solely for the purpose of stem cell research.

Hence, in a yet preferred embodiment, the terms "human embryonic stem cells" preferably refer to human embryonic stem cells isolated from discarded embryos or research embryos, advantageously by techniques that do not involve the destruction of an embryo.

According to a specific embodiment, depending on the national law, the method of the invention can be carried out with human embryonic stem cells isolated by techniques that involve the destruction of an embryo.

By **"discarded embryos"** it is herein referred to embryos specifically created in the process of an *in vitro* fertilization and declared unwanted by the human subjects it originates from.

By **"research embryos"** it is herein referred to embryos specifically created for the purpose of research or donated to scientific research.

By **"mammalian perinatal stem cells",** it is herein referred to mammalian stem cells derived from the amniotic fluid, placenta, maternal blood supply, umbilical cord and Wharton's Jelly.

By **"human perinatal stem cells",** it is herein referred to human stem cells derived from the amniotic fluid, placenta, maternal blood supply, umbilical cord and Wharton's Jelly. Such cells can thus be obtained from tissue samples rather than human embryos, the destruction of which they do not require. Those cells have been thoroughly described in Cetrulo et al. (Perinatal Stem Cells, Second Edition, Wiley-Blackwell, 2013), which the person skilled in the art may refer to.

By **"human somatic stem cells"** or "human adult stem cells" it is herein referred to stem cells found throughout the human body after birth. Such cells can thus be obtained from adult tissue samples rather than human embryos, the destruction of which they do not require.

According to the invention, **"human somatic stem cells"** encompass hematopoietic stem cells, mesenchymal stem cells, endothelial stem cells, neural stem cells, olfactory adult stem cells, neural crest stem cells, and testicular cells.

Cells derived from bone marrow and amniotic fluid, which can include both hematopoietic stem cells and mesenchymal stem cells, have been found to differentiate into beta cells with manipulation in an *in vitro* environment (Jiang et al.; Nature.; 418:41-4; 2002, and De Coppi et al.; Nat Biotechnol.;25:100-106 ; 2007).

Preferably, the term **"human somatic stem cells"** refers to hematopoietic stem cells and mesenchymal stem cells.

According to the invention, the term **"hematopoietic stem cells"** refers herein to a stem cell displaying a hematopoietic stem cells phenotype.

By "hematopoietic stem cells phenotype" it is herein meant the expression of at least one hematopoietic stem cells marker, and/or the presence of an hematopoietic stem cells morphology.

Examples of typical hematopoietic stem cells markers include, without limitation, CD34+, CD59+, Thy1/CD90+, CD38lo/-, and C-kit/CD117+.

As regards their morphology, hematopoeitic stem cells are non-adherent and rounded cells, with a rounded nucleus and low cytoplasm-to-nucleus ratio. They can further be identified by their small size, lack of lineage (lin) markers, low staining (side population) with vital dyes such as rhodamine 123 (rhodamineDULL, also called rholo) or Hoechst 33342, and presence of various antigenic markers on their surface.

Hematopoietic stem cells can be found in bone marrow and bone marrow biological samples.

According to the invention, the term **"mesenchymal stem cells"** refers herein to a stem cell displaying a mesenchymal stem cell phenotype.

By "mesenchymal stem cell phenotype" it is herein meant the expression of at least one mesenchymal stem cells marker, and/or the presence of a mesenchymal stem cell morphology.

Examples of typical mesenchymal stem cell markers include, without limitation, CD73, CD90 and CD105. Mesenchymal stem cells lack the expression of the markers CD11b, CD14, CD19, CD34, CD45, CD79a and HLA-DR.

As regards their morphology, mesenchymal stem cells are characterized by a small cell body with a few cell processes that are long and thin. The cell body contains a large, round nucleus with a prominent nucleolus, which is surrounded by finely dispersed chromatin particles, giving the nucleus a clear appearance. The remainder of the cell body contains a small amount of Golgi apparatus, rough endoplasmic reticulum, mitochondria, and polyribosomes. The cells, which are long and thin, are widely dispersed and the adjacent extracellular matrix is populated by a few reticular fibrils but is devoid of the other types of collagen fibrils.

Mesenchymal stem cells can be found for example in placenta, adipose tissue, lung, bone marrow and blood, Wharton's jelly from the umbilical cord, muscle, and teeth (perivascular niche of dental pulp and periodontal ligament).

By **"bioengineered stem cells",** it is herein referred to pluripotent stem cells artificially derived from a non-stem cell. In the context of the invention, the terms "bioengineered stem cells", encompass pluripotent stem cells obtained from somatic cell nuclear transfer (SCNT, those cells are hereafter referred to as "SCNT cells") and cells obtained from pluripotency-induced by compounds-mediated reprogramming (those cells are hereafter referred to as induced pluripotent stem cells, iPS cells or iPSCs).

Those two types of cells and techniques are well known in the art. The specific traits and advantages of each of those two types of bioengineered stem cells have particularly been described in Pan et at.(Bioessays.;34(6):472-6; 2012), which the person skilled in the art may refer to.

By **"human somatic cell nuclear transfer cells",** also abbreviated as SCNT cells, it is herein referred to pluripotent stem cells artificially derived from a non-pluripotent cell by reprogramming by somatic cell nuclear transfer.

Briefly, the nucleus of a somatic cell is removed and kept, and a host's egg cell nucleus is removed and discarded. The lone nucleus is then fused with the egg cell (lacking a nucleus). After the somatic-cell nucleus has been inserted into the egg, the egg is stimulated with a shock and will begin to divide. After several mitotic divisions, this single cell forms an artificial blastocyst (an early stage embryo with about 100 cells). SCNT cells can thus be obtained from adult tissue samples and egg cells, rather than human embryos, the destruction of which they do not require.

Examples of techniques that can be used for obtaining SCNT cells have been disclosed for example in Tachibana et al. (Cell; 153(6):1228-38; 2013) and in Markoulaki et al. (Methods; 45(2):101-14; 2008).

By **"human induced pluripotent stem cells",** also abbreviated as iPS cells or iPSCs, it is herein referred to pluripotent stem cells artificially derived from a non-pluripotent cell by compound mediated reprogramming. In the context of the invention, compound-mediated reprogramming includes for instance factor-mediated reprogramming and small-molecule compounds reprogramming.

Briefly, in factor-mediated reprogramming, iPS cells can be derived from somatic stem cells by inducing the expression of a number of specific genes, considered pluripotent related transcription factors.

Somatic cells from any tissue, such as for example stomach cells, liver cells, skin cells, blood cells, prostate cells and urinary tract cells, can be used to obtain induced pluripotent cells.

Examples of pluripotent related transcription factors include, without limitation, genes of the Oct family gene, the Klf family gene, and the Myc family gene, the Sox family gene and more particularly the genes OCT3/4, Klf1, Klf2, Klf4, Ktf5, c-Myc, N-Myc, L-Myc, Sox1, Sox2, Sox3, Sox7, Sox15, Sox17, Sox18, Nanog, and ZFP42.

Examples of techniques that can be used for obtaining iPS cells by factor-mediated reprogramming, have been disclosed for example in Loh et al. (Annu Rev Genomics Hum Genet.; 12: 165-185; 2011), EP 1 970 446, Sindhu et al. (J Biol Chem.; 287(37):30922-31; 2012), and Najm et al. (Nat Biotechnol.; 31 (5):426-3; 2013).

In small-molecule compounds reprogramming, iPS cells can be derived from somatic stem cells by specific chemicals, called small-molecule compounds.

Examples of techniques that can be used for obtaining iPS cells by factor-mediated reprogramming have been disclosed for example in Hou et al. (Science; 341 (6146):651-4, 2013).

By **"cells of the pancreatic endocrine lineage**", it is herein referred to pancreatic endocrine islet cells or progenitor thereof.

According to the invention, the terms **"endocrine islet cells"** refer to endocrine cells in the islet of Langerhans of the pancreas, and encompass alpha cells, beta cells, delta cells, pp cells, epsilon cells. In the context of the invention, the term **"endocrine islet cells"** refers herein to a cell displaying an endocrine islets cells phenotype, that is to say cell displaying alpha cells, beta cells, delta cells, pp cells, or epsilon cells phenotype.

Preferably, the terms **"endocrine islet cells"** refer to beta cells, i.e. to cell displaying beta cell phenotype.

By **"beta cell phenotype"** it is herein meant the expression of at least one beta cell marker, and/or the presence of beta cell morphology, and/or beta cell activity.

Examples of typical beta cell markers include, without limitation, IAPP, INS1/2, PCSK1, INSM1, HADH, ST18, Protein phosphatase 1, PPP1R1A, Atf2, Atf3, Atf4, Arnt/Hif, Oct1, Nfat and Stra13. Those markers and their relevance in detecting beta cells is thoroughly described in Martens et al. (PLoS One.; 6(9):e24134; 2011).

As regards their morphology, beta cells are characterized by insulin-containing granules that can easily be observed by fluorescence microscopy.

In the context of the invention, the terms "beta cell activity" refer to the production of insulin and/or C-peptide upon glucose stimulation, which can easily be assayed by techniques well known from the person skilled in the art, such as ELISA. Such techniques have for example been described in Baeyens et al. (Diabetologia; 48(1):49-57; 2005). Preferably, the terms "beta cell activity" refer to the production of insulin.

Preferably, the cell of the pancreatic endocrine lineage obtainable by a method of the invention is a functional beta cell, that is to say a cell displaying a functional beta cell phenotype.

By **"functional beta cell phenotype"** it is herein referred to a cell of the pancreatic endocrine lineage which displays insulin secretion activity upon stimulation with glucose. Such phenotype can easily be assayed by techniques well known from the person skilled in the art, such as ELISA. Such techniques have for example been described in Baeyens et al. (Diabetologia; 48(1):49-57; 2005).

According to the invention, the terms " **progenitors of endocrine islet cells"** refer to cells having the ability to differentiate into endocrine cells as previously defined, that is to say into alpha cells, beta cells, delta cells, pp cells, or epsilon cells. Preferably, in the sense of the invention, the terms "progenitors of endocrine islet cells" refer to cells having the ability to differentiate into beta cells as herein defined. In the context of the invention, the term "progenitors of endocrine islet cells" refers to a stem cell displaying the phenotype of a progenitor of endocrine islet cells, preferably to a stem cell displaying the phenotype of a progenitor of beta cells.

By "phenotype of a progenitor of endocrine islet cells" it is herein meant the expression of at least one marker of precursors of endocrine islet cells.

Examples of markers of progenitors of endocrine islet cells include, without limitation, NGN3, NKX2.2, NeuroD, ISL-1, Pax4, Pax6, or ARX. Preferably, according to the invention, a cell will be considered as a precursor of endocrine islets cells if it expresses at least the marker NGN3.

By "H₂O₂", it is herein referred to hydrogen peroxide, also called dihydrogen dioxide (IUPAC), corresponding to the CAS number 7722-84-1.

By **"precursors of H₂O₂",** it is herein referred to compounds capable of inducing H₂O₂. Precursors of H₂O₂ in the cells include for example glucose oxidase and superoxides. Preferably, according to the invention, H₂O₂ is induced by addition of glucose oxidase in the culture medium.

Superoxides, and in particular the superoxide anion radical O⁻₂, are produced in mitochondria. Superoxide dismutation leads to the production of hydrogen peroxide and dioxygen. Hydrogen peroxide and superoxides can also react together to generate highly reactive hydroxyl radicals which will oxidize some components of the cell. Superoxides can also react with nitrogen monoxide to produce peroxinitrite.

Preferably, according to the invention, the precursor of H₂O₂ is chosen in the list consisting in glucose oxidase and superoxides. Preferably, the superoxide is the superoxide anion radical O⁻₂. By the terms "is at a maximal concentration of about 30µmol/L to 90µmol/L", it is to be understood that H₂O₂ is at a maximal concentration of about 30µmol/L to 90µmol/L in the culture medium of the stem cells of the invention.

It is well known that some products, and in particular H₂O₂, are not stable under certain circumstances, which means that its concentration in the cell culture medium is susceptible to change over time.

Typically, in the sense of the invention, the terms "maximal concentration of about" refer to the concentration of product in the culture medium after addition of said product to the medium.

Preferably, the cell culture medium comprises H₂O₂ at a maximal concentration of about 40µmol/L to 80µmol/L. More preferably, the H₂O₂ maximal concentration is of about 50µmol/L to 75µmol/L. Yet preferably, the H₂O₂ maximal concentration is of about 75µmol/L.

The person skilled in the art will easily adapt the maximal concentration of precursors of H₂O₂ so as to obtain the appropriate technical effects, that is to say the same technical effects as obtained with the concentrations of H₂O₂ indicated herein.

By the terms *"in vitro* **method for obtaining cells of the pancreatic endocrine lineage from stem cells",** it is herein referred to any method known in the art that enable differentiation of stem cells, as herein described, into cells of the pancreatic endocrine lineage, and that involves a step of culturing stem cells in a cell culture medium.

Such methods include for instance the method described in D'Amour et al. (Nat Biotechnol., (11):1392-401, 2006).

Thus, according to the invention, H₂O₂ will be added to the cell culture medium used in the method of choice.

Preferably, in the *in vitro* method for obtaining beta cells of the invention, the stem cells are cultured in the cell culture medium of the invention for at least 1 hour, more preferably at least 2 hours, more preferably at least 3 hours, and even more preferably at least 4 hours.

Preferably, the stem cells used in the method of the invention are mammalian stem cells, preferably human stem cells as herein defined.

In the context of the invention, when human embryonic stem cells are used in the method of the invention, such cells are preferably human embryonic stem cells which isolation has not involved the destruction of an embryo, that is to say human embryonic stem cells isolated by techniques that do not involve the destruction of an embryo.

The terms **"cell culture medium",** "culture medium", "growth medium" or "cell medium" according to the invention encompass any medium comprising growth-promoting components, and which is suitable for the maintenance and proliferation of the cells to be cultured (i.e. grown).

Typical culture media that can be used in the method of the invention are easily available and well-known in the art. Examples of such media include, without limitation, Dulbecco's Modified Eagle's Medium@ (DMEM), DMEM F12 medium®, Eagle's Minimum Essential Medium@, F-12K medium®, Iscove's Modified Dulbecco's Medium@ and RPMI- 1640 medium®. Many media are also available as low-glucose formulations, with or without sodium pyruvate. A preferred culture medium to be used in the above method is Dulbecco's Modified Eagle's Medium@ (DMEM), and most particularly, Dulbecco's Modified Eagle's Medium@ (DMEM) High Glucose, GlutaMAX™.

Also contemplated in the present method is supplementation of the culture medium with mammalian sera, which contains cellular factors and components that can be necessary for cell maintenance and proliferation. Examples of sera include, without limitation, fetal bovine serum (FBS), bovine serum (BS), calf serum (CS), fetal calf serum (FCS), newborn calf serum (NCS), goat serum (GS), horse serum (HS), human serum, chicken serum, porcine serum, sheep serum, rabbit serum, serum replacements and bovine embryonic fluid. A particularly preferred culture medium to be used in the above method is DMEM (such as High Glucose, GlutaMAX™), supplemented with fetal bovine serum (FBS), such as 1% to 30% FBS-supplemented DMEM medium, preferably 5 to 20% FBS-supplemented DMEM medium, and most preferably 10% FBS-supplemented DMEM medium.

Some specific growth-promoting components may be specifically added to the culture medium in order to further enhance the differentiation of stem cells into the cells of interest. For example, the person skilled in the art may further complement the culture medium with components known to have an enhancing effect on differentiation, such as sodium butyrate, activin A, BMP antagonists, such as Noggin, in combination with EGF or betacellulin, nicotinamide.

Thus, in an embodiment, the cell culture medium further comprises at least one compound chosen from the group consisting of: sodium butyrate, activin A, BMP antagonists, such as Noggin, in combination with EGF or betacellulin and nicotinamide.

Preferably, incubation of the cells is performed in conventional cell culture conditions.

Cell culture techniques are fully explained in the literature, such as "Culture of Animal Cells: A Manual of Basic Technique and Specialized Applications" (2010), 6th Edition, by Freshney, and "Embryonic Stem Cell Differentiation in vitro" (1993) by Wiles.

Preferably, stems cells are cultured at an appropriate temperature. Preferably, human stem cells are cultured at a temperature comprised between 35°C and 39°C, more preferably human stem cells are cultured at a temperature comprised between 36°C and 38°C, even more preferably human stem cells are cultured at a temperature of 37°C.

In a preferred embodiment, the population of stem cells is incubated in the culture medium, while being attached to a solid support such as a tissue culture flask or plate, preferably via extracellular matrix components. Indeed, stem cells may require additional factors that encourage their attachment to a solid support. Examples of extracellular matrix components that can be used in the method of the invention are type I and type II collagen, poly-D and poly-L-tysine, polyornithine, chondroitin sulfate, fibronectin, "superfibronectin" and fibronectin-like polymers, gelatin, thrombospondin and vitronectin. Preferably, said population of cells are maintained and grown in the culture medium as defined above on a solid support coated with fibronectin, "superfibronectin" or a fibronectin-like polymer. Most preferably the solid support is coated with fibronectin at a concentration of at least 20µg/ml. The use of fibronectin may also assist in cell proliferation and differentiation.

The cells of the pancreatic endocrine lineage obtainable with a method of the invention are particularly useful for therapeutic purposes. For example, such cells can be used to replace deficient pancreatic cells in patients afflicted by a metabolic disorder, such as type I or Type II diabetes. Likewise, such cells may be used for the *in vitro* production of specific products, such as the *in vitro* production of insulin for example.

The invention thus further pertains to a cell of the pancreatic endocrine lineage obtainable by a method of the invention.

In an embodiment, the cell of the pancreatic endocrine lineage obtainable by a method of the invention is intended for its use as a medicament.

The cell of the pancreatic endocrine lineage obtainable by a method of the invention may be intended for treating or preventing a pancreatic disorder.

Thus, the present invention relates to a cell of the pancreatic endocrine lineage as defined above, for its use as a medicament for treating or preventing a pancreatic disorder

The invention is also relative to the use of a cell of the pancreatic endocrine lineage obtainable by a method of the invention for the manufacture of a medicament, preferably intended for treating or preventing a pancreatic disorder.

In addition, the invention provides a method for treating or preventing a pancreatic disorder in a human subject in need thereof, comprising administering or grafting an effective amount or number of cells of the pancreatic endocrine lineage obtainable by a method of the invention to said subject.

Such treatments are well known from the skilled person and include for instance grafting of cells in the pancreas of a subject. It has been shown that this type of therapeutic approach reduces glycemic variability and the risk for hypoglycemic events. The use of cells in such treatment has been described in Gillard et al. (Verh K Acad Geneeskd Belg.; 72(1-2):71-98; 2010) and Alipio et al. (Proc Natl Acad Sci U S A.; 107(30):13426-31; 2010).

The pancreatic disorder is preferably chosen in the group consisting of pancreatitis, such as acute pancreatitis and chronic pancreatitis, diabetes mellitus, exocrine pancreatic insufficiency (EPI), cystic fibrosis (also known as mucoviscidosis), congenital malformations, such as pancreas divisum and annular pancreas, neoplasms (such as serous cystadenoma of the pancreas, solid pseudopapillary neoplasm or Zollinger-Ellison syndrome), and Hemosuccus pancreaticus.

Preferably said pancreatic disorder is diabetes mellitus. Yet preferably, said pancreatic disorder is type I diabetes. In another embodiment, said pancreatic disorder is type II diabetes

Preferably, the cell of the pancreatic endocrine lineage obtainable by a method of the invention for treating or preventing a pancreatic disorder is a beta cell as herein defined, that is to say a stem cell displaying beta cell phenotype as herein defined. Yet preferably, the cell of the pancreatic endocrine lineage obtainable by a method of the invention for treating or preventing a pancreatic disorder is a functional beta cell as herein defined.

The invention also pertains to the use of a cell of the pancreatic endocrine lineage obtainable by a method of the invention for the *in vitro* production of insulin.

The invention further pertains to the use of a cell of the pancreatic endocrine lineage obtainable by a method of the invention for the *in vitro* identification of compounds capable of modulating insulin production.

By the terms **"capable of modulating"** insulin production, it is herein referred to compounds capable of increasing or decreasing the amount of insulin produced *in vitro* by cells of the pancreatic endocrine lineage obtainable by a method of the invention.

Thus, the invention is also relative to a method of screening compound that modulates insulin production comprising the step of:
a) contacting a cell obtained by the method according to the present invention with a test compound;
b) determining the level of insulin.

Preferably, said method includes a further step of comparing the level of insulin to an insulin control level.

Preferably, the insulin control level correspond to the one measured in the same conditions than the one of step a), but without the presence of the test compound. Preferably, the insulin control level is measured before step a).

Preferably, said method includes a further step of concluding that the test compound increases the insulin production, if the level of insulin is higher than the insulin control level.

Preferably, the one skilled in the art will conclude that the test compound increases the insulin production, if the level of insulin is at least 20%, preferably at least 30%, more preferably at least 50% and even more preferably at least 70% higher than the insulin control level.

Preferably, said method includes a further step of concluding that the test compound decreases the insulin production, if the level of insulin is lower than the insulin control level.

Preferably, the one skilled in the art will conclude that the test compound decreases the insulin production, if the level of insulin is at least 20%, preferably at least 30%, more preferably at least 50% and even more preferably at least 70% lower than the insulin control level.

### EXAMPLES

### Experimental Procedures.

### Animals

Pregnant Wistar Rats were purchased from the Janvier Breeding center (CERJ Janvier, Le Genest Saint-Ile, France). The animals had free access to food pellets and water. Embryonic day 0.5 (E0.5) was considered the first day postcoïtum. Pregnant female were killed by CO₂ asphyxiation according to the guidelines of the French Animal Care Committee.

N-Acetyl cystein (Sigma, Saint-Quentin Fallavier, France) treatment was initiated during embryonic life at E13.5 and given into drinking water to pregnant females.

### Pancreas dissection and culture

The embryos were harvested on E13.5. The dorsal pancreatic bud was dissected, as described previously (Attali et al., 2007). For the depletion of the mesenchyme, the digestive tract was incubated with 0.5 mg/ml collagenase A (Roche, Meyaln, France) at 37°c for 30 min, then washed several times with Hank's balanced solution (Invitrogen, Cergy-Pontoise, France) at 4°c, and the epithelium was mechanically separated from the mesenchyme using needles on 0.25% agar, 25% Hank's balanced solution and 75% RPMI gel (Life Technologies, Invitrogen) in a petri dish. Pancreatic rudiments (epithelium with mesenchyme) or epithelia were then cultured at the air-medium interface on Millicell culture plate inserts (Millipore, Molsheim, France) in Petri dishes containing RPMI 1640 (Invitrogen) supplemented with penicillin (100 U/ml), streptomycin (100 g/ml), HEPES (10 mM), l-glutamine (2 mM), nonessential amino acids (1; Invitrogen, Cergy-Pontoise, France) and 10% heat-inactivated calf serum (Hyclone, South Logan, UT, USA) for one to 7 days. H₂O₂ (Sigma) was used at 50, 75, 100 □mol/l. Glucose (Sigma, Saint-Quentin Fallavier, France) was used at 20 mM/l and the inhibitor of the ERK pathway (U0126, Cell Sinaling Technologies Ozyme, Saint-Quentin, France) at 10 µmol/l. Cultures were maintained at 37°C in humidified 95% air and 5% CO2.

For proliferation measurment, BrdU was added during the last hour of culture (10 µmol/l).

### Infection

Ad-CMV-GFP (ref 1060, Vector Biolabs, Philadelphia, PA, USA) and Ad-Catalase (ref 1475, Vector Biolabs) adenoviruses were used to infect the embryonic Wistar rat epithelia. Tissues were incubated with viral particles at the multiplicity of infection of 1000 for 4 h at 37°C in RPMI 1640 as described in (Heinis et al.2012). After infection, tissues were washed twice in HBSS and cultured at the air-medium interface.

### Immnuhistochemistry

Tissues were fixed in 10% formalin and processed for immunohistochemistry, as described previously (Attali et al., 2007). The following antibodies were used: mouse anti-insulin (1:2000; Sigma-Aldrich), rabbit anti-PDX1 (1:1,000), rabbit anti-amylase (Sigma-Aldrich, 1:300), mouse anti-E-cadherin (1:100, BD Biosciences, Le Pont de Claix, France), mouse anti-BrdU (1:2, Amersham, Velizy-Villacoublay, France), rabbit anti-NGN3 (1:1000) (Guillemain et al., 2007), rabbit anti-catalase (1:1000, Rockland Immunochemicals, Gilbertsville, PA USA). The fluorescent secondary antibodies were fluorescein-isothiocyanate anti-rabbit and Texas-red anti-mouse antibodies (1:200, Jackson Immunoresearch, Suffolk, England), and Atexa-fluor anti-rabbit antibody (1:400, Biogenex, Fremont, CA, USA). For NGN3, revelation was performed using the vectastain ABC kit (Vector LAB, Peterborough, England). Photographs were taken using a fluorescence microscope (Leitz DMRB; Leica) and digitized using a Hamamatsu (Middlesex, NJ, USA) C5810 cooled 3CCD camera.

### Transferase-mediated dUTP nick-end labeling experiments

The TUNEL procedure was performed using an in situ cell death detection kit (Roche, Boulogne-Billancourt, France) according to the manufacturer's instructions. Subsequently, E-cadherin (1/100, BD Biosciences, Rungis, France) immunostaining was used to visualize the epithelium.

### Real-Time PCR

Total RNA was purified using the Rneasy microkit (Qiagen, Courtaboeuf, France). The cDNA was generated using Superscript reagents (Invitrogen), and the real-time PCR was performed on a 7300 real-time PCR system (Applied Biosystem, Invitrogen) with an SYBR Green PCR master mix. The oligonucleotide sequences for RT-PCR are available on request.

### H₂O₂ stability measurement

Residual hydrogen peroxide was quantified by polarographic determination of the oxygen released upon addition of 10 µg of purified beef liver catalase resuspended in 50 mM phosphate buffer pH 7.0 (Sigma) to 250 µl culture medium samples placed in an oxygen polarograph (Hansatech, Norfolk, England).

### Western Blot Analysis

For Western blotting analysis, cells were lysed in Laemmli buffer. Proteins (20µg) were resolved by SDS/PAGE and electrophoretically transferred onto PVDF membrane (Bio-Rad, Hercules, CA, USA). After blocking with milk, membranes were probed with rat anti-p44/42 MAPK (Erk1/2), mouse anti-Phospho-p44/42 MAPK (Erk1/2) (Thr202/Tyr204), anti-Phospho-MEK (all from Cell Signaling Ozyme, ST Quentin en Yvelines, France) and mouse anti-actin (Sigma-Aldrich).Immunoreactive bands were visualized with the SuperSignal System (Pierce; Fisher Scientific, Illkirch, France).

### Quantification

To quantify the absolute number of insulin-expressing cells, all sections of each pancreatic rudiment were digitized. On every image, the surface area of insulin staining was quantified using ImageJ as described in (Heinis et al.).

To quantify the number of NGN3-positive cells, NGN3-positive cells were counted on all sections. Statistical significance was determined using Student's t test.

To measure the proliferation of early progenitors expressing E-cadherin, we counted the frequency of BrdU+ nuclei among 1000 PDX1+ cells. Three rudiments per condition were analyzed. Statistical significance was determined using Student's t test.

To measure cell survival, we counted the frequency of TUNEL+ cells among E-cadherin+ cells. Statistical significance was determined using Student's t test.

### Results.

### 1 /ROS are required for normal beta cell development in vivo

Female pregnant rats at 13.5 days postcoitus were treated with NAC, and the number of NGN3-positive cells was measured.

The number of NGN3-positive cells was 1.6-fold decreased in the embryonic pancreases from fetuses derived from pregnant rats treated with NAC compared to controls (Fig. 1A).

2.5-fold reduction of the beta cell mass at E20.5 in pancreases treated with NAC was also obsered (Fig. 1 B).

Together, these results indicate that ROS are implicated in beta cell differentiation. 2/ROS enhance beta cell differentiation in embryonic pancreases

E13.5 rat embryonic pancreases were cultured with various concentrations of H₂O₂. The decay of H₂O₂ over time was measured using a polarograph (Dempsey et al., 1975) (Figure 9). He results show that H₂O₂ lasts for 4 hours in the culture medium. Next, pancreatic explants were treated with H₂O₂ and were analyzed seven days later (Figure 2A-B). When H₂O₂ was added at a concentration of 50µM, the development of beta cells was increased, the insulin surface being 2.6-fold larger than controls.

The mechanism by which H₂O₂ increases beta cell development was next examined. 3 hypothesis were tested:
1 /H₂O₂ could increase the survival or
2/ H₂O₂ could increase the proliferation of the progenitor cells, or
3/ H₂O₂ could either amplify their differentiation into endocrine cells.

The rate of apoptosis was quantified using a TUNEL reaction assay. Cells were tested after one day of culture in the presence or in the absence of H₂O₂. No difference was observed between the H₂O₂ treated pancreases and controls (Figure 3A).

BrdU incorporation was measured, to evaluate cell proliferation in pancreases cultured with or without H₂O₂ (Figure 3B). Again, the rate of cell proliferation was similar in the two groups of tissues. Finally, it was researched whether H₂O₂ has the ability to enhance beta cell differentiation. For this, NGN3 protein expression was quantified using immunohistochemistry. The number of NGN3-positive cells was increased by nearly 2-fold in pancreases cultured with H₂O₂ (Figure 3C). These results show that H₂O₂ controls beta cell differentiation. To confirm this result, pancreases were cultured with Glucose oxidase (GOx), an enzyme which oxidizes glucose into H₂O₂ and D-glucono-lactone. In the presence of GOx, H₂O₂ is continuously generated in the medium. We found that the number of NGN3-positive cells was 2.2-fold increased in the presence of GOx compared to controls (Figure 10). These data thus demonstrate that ROS have a positive and non-toxic effect on beta cell development when applied at specific doses and duration.

Decreasing ROS production with Catalase alters NGN3 expression.

In order to control H202 production, we focused on Catalase, which catalyzes the degradation of H₂O₂ into water and oxygen. Indeed, manipulating the level of Catalase expression allows to control the level of H202 during pancreas development. Catalase protein level was increased by using both parmacological and genetical methods. E13.5 pancreases were first cultured for one day with exogenous Catalase. We observed a 3.3 fold decrease in the number of NGN3-expressing cells compared to controls (Figure 4A). Consistent with this result, we also observed a 1.75-fold reduction of the insulin staining surface in pancreases treated with Catalase (Figure 4B). These results show that exogenous Catalase prevents beta cell differentiation in the developing pancreas.

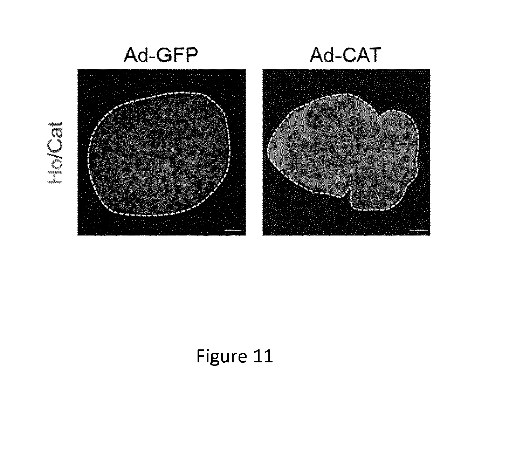

We next increased Catalase expression using adenoviral transfection. E13.5 pancreatic epithelia were infected with adenoviruses coding either for Catalase (Ad-Cat), or for the Green Fluorescent Protein (Ad-GFP, control). To check the efficiency of Ad-Cat, we detected Catalase protein expression using immunohistochemistry. Catalase expression (Figure 11) was found in the majority of pancreatic epithelial cells. As expected, pancreases infected with Ad-GFP were all negative for Catalase protein expression. Infection with Ade-Cat did not modify epithelial progenitor cell apoptosis measured by TUNEL assay, nor progenitors proliferation measured by the quantification of BrdU incorporation (Figure 5A-B). On the other hand, the number of NGN3-positive endocrine progenitor cells was decreased by 1.6-fold in pancreases infected by Ad-Cat compared to pancreases infected by Ad-GFP(Figure 5C). Finally, beta cell development was decreased in pancreases infected with Ad-Cat versus controls (Figure 5D). These results thus confirm the previous results with exogenous Catalase. Altogether, these data show that the level of Catalase regulates beta cell development.

### 3/ROS signals which control beta cell development have a mitochondrial origin

To further confirm the implication of ROS in beta cell differentiation, embryonic pancreases were cultured in the presence of the antioxydant NAC, or with the mitochondrial decoupling agent Carbonyl cyanide m-chlorophenyl hydrazone (CCCP). Indeed, these compounds are known to decrease ROS levels. Both NAC and CCCP treatments decreased nearly 2-fold the number of NGN3-positive endocrine progenitor cells (Figure 6A). Such reductions were followed by a decrease of the insulin staining surface at day 7 (Figure 6B).

In conclusion, endogenous ROS are mandatory for beta cells to differentiate. Moreover, the effects obtained with CCCP indicate that in this process, ROS have a mitochondrial origin.

### 4/ROS regulation of beta cell development depends on ERK1/2 Signaling

The molecular mechanisms by which H₂O₂ increased beta-cell development was next characterized. The Notch Signaling Pathway, which is known to regulate beta-cell development was considered. Rat embryonic pancreases (E13.5) were cultured for one day with or without H₂O₂ and expression of genes encoding the Notch receptors 1 and 2, the Notch target Hes 1, the Notch ligands Jagged 1, Jagged 2, Delta-like 1 were investigated by RT-qPCR. No difference was found between H₂O₂-treated pancreases and controls (Figure 7). These data suggest that the Notch Pathway is not modulated by H₂O₂ in the embryonic pancreas.

The Mitogen Activated Protein Kinase (MAPK) ERK1/2 pathway, which was recently shown to be sensitive to ROS production in several cell types (Zhang et al., 2013) was examined. Pancreases were first cultured with or without H₂O₂ (50 µM) for 5 or 15 minutes. As a positive control, pancreases were also treated with 20 mM Glucose, an inducer of the ERK1/2 phosphorylation (Briaud et al., 2003). Western blot analysis showed that H₂O₂ induced the ERK1/2 pathway (Figure 8A). Similar results were obtained with glucose (Figure 8A). These data thus indicate that H₂O₂ is an activator of ERK1/2 in the embryonic pancreas. To further characterize ERK1/2-dependent effect of ROS on pancreas development, an ERK1/2 inhibitor was used (U0126). To validate the efficiency of the inhibitor, pancreatic explants were first cultured with glucose, in the presence or in the absence of U0126. Activation of ERK1/2 by glucose was abolished in the presence of U0126 (Figure 8A), confirming the efficiency of U0126.

The ERK1 /2-activation by H₂O₂ in the presence or in the absence of U0126 were then compared. In the presence of U0126, the activation of the ERK1/2 pathway by H₂O₂ was blunted (Figure 8A), confirming previous results.

Finally, the possibility that ERK1/2 activation by H₂O₂ is responsible for the induction of beta-cell differentiation was investigated. As previously shown, treatment of pancreases with H₂O₂ increased the number of Ngn3 expressing cells (Figure 8B). This effect was abrogated in the presence of U0126 (Figure 8B). Altogether, these results show that an intact ERK1/2 pathway is necessary for the effect of H₂O₂ on beta cell differentiation.

To determine whether the effect of H₂O₂ on beta cell development is mediated by the ERK1/2 pathway, pancreases were cultured with or without H₂O₂, in the presence or in the absence of an inhibitor of ERK1/2 (U0126). In the presence of U0126, the activation of the ERK pathway by H₂O₂ was blunted (Figure 8B), confirming previous results. In order to determine whether H₂O₂ controls beta cell differentiation through ERK1/2, pancreases were cultured with H₂O₂, in the presence or in the absence of U0126. As previously shown, treatment of pancreases with H₂O₂ increased the number of NGN3 expressing cells (Figure 8B). This effect was abrogated in the presence of U0126 (Figure 8B). Altogether, these results show that an intact ERK1/2 pathway is necessary for the effect of H₂O₂ on beta cell differentiation.

### Conclusion

The present results demonstrate that both exogenous and endogenous ROS can have a significant impact on pancreatic progenitor cell differentiation. Moreover, these beta cells generated by ROS-treatment are functional as they secrete insulin in response to glucose.

This is surprising as ROS have been implicated in toxic effects on beta cells development in other models. For example, uteroplacental insufficiency in rats leads to intrauterine growth retardation (IUGR) and disrupts the function of the electron transport chain in the fetal b-cell. This process induces increased production of ROS which in turn damage mitochondrial DNA and causes further production of ROS (Simmons, 2007). These events cause a progressive loss of beta cells function and type-2 diabetes in the adult. This model of IUGR mimics Type2 diabetes in humans, with progressive defects in insulin secretion and insulin action prior to hyperglycemia.

A difference between these experiments and the present study, is that the increase of ROS occurred in differentiated beta cells in the IUGR model, downstream of NGN3 expression, meanwhile we manipulated ROS signals upstream of NGN3 expression in our study. Another possibility is that ROS are not the only cause of beta cell defects in the IUGR model. Further studies will be necessary to elucidate these mechanisms.

### ROS and MAP Kinase signaling.

In our culture experiments, beta cell differentiation was induced by an initial pulse of H₂O₂. Indeed, when H₂O₂ was added at the beginning of the culture, it lasted only for four hours in the medium (Figure 9). On the basis of these results, it is expected to observe a rapid effect of H₂O₂ on intracellular signalization within the progenitor cells. Consistent with this hypothesis, ERK1/2 activation was observed only five minutes after the addition of H₂O₂. This finding suggests that rapid variations of H₂O₂ control the ERK1/2 pathway in the pancreatic progenitors. Moreover, using a ERK1/2 inhibitor, it was showed that an intact ERK1 /2 pathway is necessary for the effects of H₂O₂ on endocrine differentiation. In our previous work, we also found that activation of the MAP kinase/ERK1/2 pathway by Fibroblast Growth Factor 10 (FGF10) controls the development of beta cells (Attali et al., 2007; Bhushan et al., 2001). Such data reinforces our conclusion on the role of ERK1/2 in the development of beta cells and indicate that ERK1/2 pathway is controlled by different signals in the embryonic pancreas. Finally, ROS were shown to be needed for the activation of three MAP Kinases, ERK, JNK, and p38, at early stage of monocyte-macrophage differentiation. Late ERK activation is also essential for monocyte-macrophage differentiation (Zhang et al., 2013). We thus speculate that oscillations of H₂O₂ production and activation of ERK1/2 may have a role in a range of different types of progenitor cells.

The effects of ROS on stem cell or progenitor cell differentiation is context dependent.

A role of ROS has been suggested in the differentiation of embryonic stem cells, iPS, and some adult stem cells as hematopoietic stem cells, adipocytes, and neural stem cells (Miyamoto et al., 2007; Naka et al., 2010; Renault et al., 2009; Yalcin et al., 2008).

Recently, it was shown that both pluripotent and multipotent stem cells possess enzymatic and non enzymatic mechanisms for detoxification of ROS and to correct oxidative damage to the genome as well as the proteome. Interestingly, a correlation exists between antioxydant defense level and stem cell change, i.e proliferation, differentiation and death (Chaudhari et al., 2012).

Despite the broad influence of ROS-mediated signaling on cell differentiation, there are many cases in which cellular response to ROS depends on other factors, such as cell phenotype, cell differentiation state and the presence of cell-specific transcription factors (Le Belle, 2011). Indeed, it was shown that H₂O₂ can activate other signaling pathways as SIRT1 deacetylase, calcium influx, Nuclear-Factor E2 related Factor 2 (Nrf2), PI3Kinase/Akt (Brunet et al., 2004; Kraft et al., 2004a; Kraft et al., 2004b; Le Belle et al., 2011), or can induce genotoxic stress that may induce differentiation of other cell types as for example melanocytes (Inomata et al., 2009). It is thus possible that one of these pathways is involved in beta cell differentiation. The present results show that the effects of ROS on beta cell differentiation require the activation of P-ERK1/2 signaling pathway. When analysing the expression of Nrf2 in pancreases treated with H₂O₂, a slight increase of Nrf2 RNA levels was observed (data not shown).

### References

Arana, L., Gangoiti, P., Ouro, A., Rivera, I. G., Ordonez, M., Trueba, M., Lankalapalli, R. S., Bittman, R., and Gomez-Munoz, A. Generation of reactive oxygen species (ROS) is a key factor for stimulation of macrophage proliferation by ceramide 1-phosphate. (2012) Exp Cell Res 318, 350-360.
Arda, H. E., Benitez, C. M., and Kim, S. K. Gene regulatory networks governing pancreas development. (2013) Dev Cell 25, 5-13.
Attali, M., Stetsyuk, V., Basmaciogullari, A., Aiello, V., Zanta-Boussif, M. A., Duvillie, B., and Scharfmann, R. (2007). Control of beta-cell differentiation by the pancreatic mesenchyme. Diabetes 56, 1248-1258.
Benitez, C. M., Goodyer, W. R., and Kim, S. K. (2012) Deconstructing pancreas developmental biology. Cold Spring Harb Perspect Biol 4.
Bhushan, A., Itoh, N., Kato, S., Thiery, J. P., Czernichow, P., Bellusci, S., and Scharfmann, R. (2001). Fgf10 is essential for maintaining the proliferative capacity of epithelial progenitor cells during early pancreatic organogenesis. Development 128, 5109-5117.
Blondeau, B., Lesage, J., Czernichow, P., Dupouy, J. P., and Breant, B. (2001). Glucocorticoids impair fetal beta-cell development in rats. Am J Physiol Endocrinol Metab 281, E592-599.
Briaud, I., Lingohr, M. K., Dickson, L. M., Wrede, C. E., and Rhodes, C. J. (2003). Differential activation mechanisms of Erk-1/2 and p70(S6K) by glucose in pancreatic beta-cells. Diabetes 52, 974-983.
Brownlee, M. (2001). Biochemistry and molecular cell biology of diabetic complications. Nature 414, 813-820.
Brunet, A., Sweeney, L. B., Sturgill, J. F., Chua, K. F., Greer, P. L., Lin, Y., Tran, H., Ross, S. E., Mostoslavsky, R., Cohen, H. Y., et al. (2004). Stress-dependent regulation of FOXO transcription factors by the SIRT1 deacetylase. Science 303, 2011-2015.
Chaudhari, P., Ye, Z., and Jang, Y. Y. (2012) Roles of Reactive Oxygen Species in the Fate of Stem Cells. Antioxid Redox Signal.
D'Autreaux, B., and Toledano, M. B. (2007). ROS as signalling molecules: mechanisms that generate specificity in ROS homeostasis. Nat Rev Mol Cell Biol 8, 813-824.
Del Guerra, S., Lupi, R., Marselli, L., Masini, M., Bugliani, M., Sbrana, S., Torri, S., Pollera, M., Boggi, U., Mosca, F., et al. (2005). Functional and molecular defects of pancreatic islets in human type 2 diabetes. Diabetes 54, 727-735.
Dempsey, P. M., O'Leary, J., and Condon, S. (1975). Polarographic assay of hydrogen peroxide accumulation in microbial cultures. Appl Microbiol 29, 170-174.
Gradwohl, G., Dierich, A., LeMeur, M., and Guillemot, F. (2000). neurogenin3 is required for the development of the four endocrine cell lineages of the pancreas. Proc Natl Acad Sci U S A 97, 1607-1611.
Guillemain, G., Filhoulaud, G., Da Silva-Xavier, G., Rutter, G. A., and Scharfmann, R. (2007). Glucose is necessary for embryonic pancreatic endocrine cell differentiation. J Biol Chem 282, 15228-15237.
Heinis, M., Simon, M. T., Ilc, K., Mazure, N. M., Pouyssegur, J., Scharfmann, R., and Duvillie, B. Oxygen tension regulates pancreatic beta-cell differentiation through hypoxia-inducible factor 1alpha. Diabetes 59, 662-669.
Heinis, M., Soggia, A., Bechetoille, C., Simon, M. T., Peyssonnaux, C., Rustin, P., Scharfmann, R., and Duvillie, B. HIF1alpha and pancreatic beta-cell development. Faseb J 26, 2734-2742.
Hunt, J. V., Smith, C. C., and Wolff, S. P. (1990). Autoxidative glycosylation and possible involvement of peroxides and free radicals in LDL modification by glucose. Diabetes 39, 1420-1424.
Inomata, K., Aoto, T., Binh, N. T., Okamoto, N., Tanimura, S., Wakayama, T., Iseki, S., Hara, E., Masunaga, T., Shimizu, H., and Nishimura, E. K. (2009). Genotoxic stress abrogates renewal of melanocyte stem cells by triggering their differentiation. Cell 137, 1088-1099.
Kraft, A. D., Johnson, D. A., and Johnson, J. A. (2004a). Nuclear factor E2-related factor 2-dependent antioxidant response element activation by tert-butylhydroquinone and sulforaphane occurring preferentially in astrocytes conditions neurons against oxidative insult. J Neurosci 24, 1101-1112.
Kraft, R., Grimm, C., Grosse, K., Hoffmann, A., Sauerbruch, S., Kettenmann, H., Schultz, G., and Harteneck, C. (2004b). Hydrogen peroxide and ADP-ribose induce TRPM2-mediated calcium influx and cation currents in microglia. Am J Physiol Cell Physiol 286, C129-137.
Le Belle, J. E., Orozco, N. M., Paucar, A. A., Saxe, J. P., Mottahedeh, J., Pyle, A. D., Wu, H., and Kornblum, H. I. Proliferative neural stem cells have high endogenous ROS levels that regulate self-renewal and neurogenesis in a P13K/Akt-dependant manner. (2011) Cell Stem Cell 8, 59-71.
Lee, H., Lee, Y. J., Choi, H., Ko, E. H., and Kim, J. W. (2009). Reactive oxygen species facilitate adipocyte differentiation by accelerating mitotic clonal expansion. J Biol Chem 284, 10601-10609.
Leloup, C., Tourrel-Cuzin, C., Magnan, C., Karaca, M., Castel, J., Carneiro, L., Colombani, A. L., Ktorza, A., Casteilla, L., and Penicaud, L. (2009). Mitochondrial reactive oxygen species are obligatory signals for glucose-induced insulin secretion. Diabetes 58, 673-681.
Lenzen, S., Drinkgern, J., and Tiedge, M. (1996). Low antioxidant enzyme gene expression in pancreatic islets compared with various other mouse tissues. Free Radic Biol Med 20, 463-466.
Miyamoto, K., Araki, K. Y., Naka, K., Arai, F., Takubo, K., Yamazaki, S., Matsuoka, S., Miyamoto, T., Ito, K., Ohmura, M., et al. (2007). Foxo3a is essential for maintenance of the hematopoietic stem cell pool. Cell Stem Cell 1, 101-112.
Naka, K., Hoshii, T., Muraguchi, T., Tadokoro, Y., Ooshio, T., Kondo, Y., Nakao, S., Motoyama, N., and Hirao, A. TGF-beta-FOXO signalling maintains leukaemia-initiating cells in chronic myeloid leukaemia. (2010) Nature 463, 676-680.
Panieri, E., Gogvadze, V., Norberg, E., Venkatesh, R., Orrenius, S., and Zhivotovsky, B. Reactive oxygen species generated in different compartments induce cell death, survival, or senescence. (2013) Free Radic Biol Med 57, 176-187.
Renault, V. M., Rafalski, V. A., Morgan, A. A., Salih, D. A., Brett, J. O., Webb, A. E., Villeda, S. A., Thekkat, P. U., Guillerey, C., Denko, N. C., et al. (2009). FoxO3 regulates neural stem cell homeostasis. Cell Stem Cell 5, 527-539.
Rhee, S. G., Kang, S. W., Jeong, W., Chang, T. S., Yang, K. S., and Woo, H. A. (2005). Intracellular messenger function of hydrogen peroxide and its regulation by peroxiredoxins. Curr Opin Cell Biol 17, 183-189.
Sakuraba, H., Mizukami, H., Yagihashi, N., Wada, R., Hanyu, C., and Yagihashi, S. (2002). Reduced beta-cell mass and expression of oxidative stress-related DNA damage in the islet of Japanese Type II diabetic patients. Diabetologia 45, 85-96.
Sato, A., Okada, M., Shibuya, K., Watanabe, E., Seino, S., Narita, Y., Shibui, S., Kayama, T., and Kitanaka, C. (2013) Pivotal role for ROS activation of p38 MAPK in the control of differentiation and tumor-initiating capacity of glioma-initiating cells. Stem Cell Res 12, 119-131.
Seymour, P. A., Freude, K. K., Tran, M. N., Mayes, E. E., Jensen, J., Kist, R., Scherer, G., and Sander, M. (2007). SOX9 is required for maintenance of the pancreatic progenitor cell pool. Proc Natl Acad Sci U S A 104, 1865-1870.
Shah, S. R., Esni, F., Jakub, A., Paredes, J., Lath, N., Malek, M., Potoka, D. A., Prasadan, K., Mastroberardino, P. G., Shiota, C., et al. Embryonic mouse blood flow and oxygen correlate with early pancreatic differentiation. Dev Biol 349, 342-349.
Simmons, R. A. (2007). Role of metabolic programming in the pathogenesis of beta-cell failure in postnatal life. Rev Endocr Metab Disord 8, 95-104.
Tiedge, M., Lortz, S., Drinkgern, J., and Lenzen, S. (1997). Relation between antioxidant enzyme gene expression and antioxidative defense status of insulin-producing cells. Diabetes 46, 1733-1742.
Waris, G., and Ahsan, H. (2006). Reactive oxygen species: role in the development of cancer and various chronic conditions. J Carcinog 5, 14.
Yalcin, S., Zhang, X., Luciano, J. P., Mungamuri, S. K., Marinkovic, D., Vercherat, C., Sarkar, A., Grisotto, M., Taneja, R., and Ghaffari, S. (2008). Foxo3 is essential for the regulation of ataxia telangiectasia mutated and oxidative stress-mediated homeostasis of hematopoietic stem cells. J Biol Chem 283, 25692-25705.
Zhang, Y., Choksi, S., Chen, K., Pobezinskaya, Y., Linnoila, I., and Liu, Z. G. ROS play a critical role in the differentiation of alternatively activated macrophages and the occurrence of tumor-associated macrophages. (2013) Cell Res 23, 898-914.

## Claims

1. *In vitro* method for obtaining cells of the pancreatic endocrine lineage, comprising a step of culturing stem cells in a cell culture medium comprising H₂O₂ or a precursor thereof, wherein said stem cells exclude human embryonic stem cells isolated by techniques that involve the destruction of an embryo.

2. The method according to claim 1, wherein said cell culture medium comprises H₂O₂ at a maximal concentration from 30µmol/L to 90µmol/L.

3. The method according to claim 1 or 2, wherein said stem cells are chosen in the group consisting of embryonic stem cells, perinatal stem cell, somatic stem cells, and bioengineered stem cells.

4. The method according to any of the previous claims, wherein said stem cells are human stem cells.

5. The method according to any of the previous claims wherein said stem cells are cultured in said cell culture medium for at least one hour, preferably for at least two hours, more preferably for three hours.

6. A cell of the pancreatic endocrine lineage obtainable by a method according to any of the previous claims.

7. A cell of the pancreatic endocrine lineage according to claim 6, for use as a medicament.

8. A cell of the pancreatic endocrine lineage according to any of claim 6 or 7, for its use as a medicament for treating or preventing a pancreatic disorder, preferably chosen in the list consisting of pancreatitis, such as acute pancreatitis and chronic pancreatitis, diabetes mellitus, exocrine pancreatic insufficiency (EPI), cystic fibrosis (also known as mucoviscidosis), congenital malformations, such as pancreas divisum and annular pancreas, neoplasms (such as serous cystadenoma of the pancreas, solid pseudopapillary neoplasm or Zollinger-Ellison syndrome), and Hemosuccus pancreaticus.

9. A cell of the pancreatic endocrine lineage for the use according to claim 8, wherein said pancreatic disorder is type I diabetes.

10. *In vitro* use of H₂O₂ or a precursor thereof for obtaining cells of the pancreatic endocrine lineage from stem cells.

11. Use of a cell of the pancreatic endocrine lineage obtainable by a method of any one of claims 1 to 5 for the *in vitro* production of insulin.

12. Use of a cell of the pancreatic endocrine lineage obtainable by a method of any one of claims 1 to 5 for the *in vitro* identification of compounds capable of modulating insulin production.
